(19) **Europäisches Patentamt / European Patent Office / Office européen des brevets**

(11) **EP 1 652 498 B1**

(12) **EUROPEAN PATENT SPECIFICATION**

(45) Date of publication and mention of the grant of the patent:
**27.07.2011 Bulletin 2011/30**

(21) Application number: **04748210.4**

(22) Date of filing: **03.08.2004**

(51) Int Cl.:
*A61N 1/36* (2006.01)  *H04R 25/00* (2006.01)

(86) International application number:
**PCT/JP2004/011077**

(87) International publication number:
**WO 2005/013870 (17.02.2005 Gazette 2005/07)**

(54) **METHOD OF SPEECH CONVERSION FOR A COCHLEAR IMPLANT AND COCHLEAR IMPLANT**

VERFAHREN ZUR SPRACHUMWANDLUNG FÜR EIN COCHLEA-IMPLANTAT UND COCHLEA-IMPLANTAT

PROCÉDÉ DE CONVERSION VOCALE POUR UN IMPLANT COCHLÉAIRE ET IMPLANT COCHLÉAIRE

(84) Designated Contracting States:
**AT BE BG CH CY CZ DE DK EE ES FI FR GB GR HU IE IT LI LU MC NL PL PT RO SE SI SK TR**

(30) Priority: **07.08.2003 JP 2003206405**

(43) Date of publication of application:
**03.05.2006 Bulletin 2006/18**

(73) Proprietor: **Hamamatsu Foundation for Science and Technology Promotion Hamamatsu-shi, Shizuoka 432-8561 (JP)**

(72) Inventors:
• **Kitazawa, S.**
**c/o Shizuoka Univ. Hamamatsu Campus Hamamatsu-shi Shizuoka 432-8561 (JP)**
• **Kiriyama, Shinya**
**Shizuoka Univ. Hamamatsu Campus Hamamatsu-shi Shizuoka 432-8561 (JP)**
• **Dashtseren, E.**
**Shizuoka Univ. Hamamatsu Campus Hamamatsu-shi Shizuoka 432-8561 (JP)**
• **Iwasaki,Satoshi**
**Hamamatsu Univ. School of Medicine Hamamatsu-shi Shizuoka 431-3192 (JP)**

(74) Representative: **Cohausz & Florack Patent- und Rechtsanwälte Partnerschaftsgesellschaft Bleichstraße 14 40211 Düsseldorf (DE)**

(56) References cited:
**WO-A-01/13991        WO-A1-01/99470
JP-A- 2003 507 143      JP-A- 2003 521 337**

• **PHILIPOS L LOIZOU: "Mimicking the human ear: An Overview of Signal-Processing Strategies for Converting Sound into Electrical Signals in Cochlear Implants" IEEE SIGNAL PROCESSING MAGAZINE, September 1998 (1998-09), pages 101-130, XP011089814**

**Description**

BACKGROUND OF THE INVENTION

Field of the Invention

The present invention relates to a method of speech conversion in a cochlear implant (artificial cochlear) for reinforcing the acoustic sense by providing the speech information to the cochlea as electrically stimulating pulses.

Description of Background Art

Heretofore, the cochlear implant is provided with an electrode array of a plurality of electrodes arranged within a cochlea, and discriminates speech information sampled via a microphone into respective frequency bands using a plurality of band pass filters. Thus the acoustic sense is reinforced by transmitting speech information in respective channels to the electrodes corresponding to the channels to generating electrically stimulating pulses and by transmitting the speech information to the cochlea as electrically stimulating pulses. Concretely, the speech conversion is carried out by converting the speech information to the stimulating pulses using a speech processor arranged within the cochlear implant. In such a cochlear implant, it is impossible to obtain a faithful sound sense relative to actually processed speech signals when the electrodes are simultaneously stimulated. This is because of generation of unsuitable stimulation caused by mutual action between electrode circuits when the electrodes are simultaneously stimulated.

For resolving such a problem, a continuous interleaved sampler (CIS) system is adopted as the conventional speech converting method in the cochlear implant. In this system, all channels are successively stimulated at a predetermined interval in order to avoid the simultaneous stimulation of channels and to output all speech information without any omission. That is, no speech information can be outputted until it arrives at its order although it is generated simultaneously with any other speech information.

Since the CIS is not a known invention disclosed in any publication, there is no information relating to publications of the prior art.

According to the CIS system of the prior, since all channels are successively stimulated at a predetermined interval, the speech would sometimes be outputted at a timing delayed from that at which the speech is actually generated and thus the regenerated speech would be unnatural.

The article "Mimicking the human ear: An Overview of Signal-Processing Strategies for converting sound into Electrical signals in Cochlear Implants", Philipos L Loizou, IEEE SIGNAL PROCESSING MAGAZINE, September 1998, discloses transmitting in formation of only six channels to the electrodes of a clochlear implant within an interval of 4ms.

SUMMARY OF THE INVENTION

It is, therefore, an object of the present invention to provide a cochlear implant and a method speech conversion in the cochlear implant which can provide a user with a speech of almost natural feeling and can regenerate a natural variation at a value near the natural period of respective channels.

According to the invention, there are provided a method as set forth in claim 1 and a cochlear implant as set forth in claim 2.

Although a term "speech" is used in the description of the specification, it should be noted that it includes not only a voice of human but any other audible sounds.

BRIEF DESCRIPTION OF THE DRAWINGS

Additional advantages and features of the present invention will become apparent from the subsequent description and the appended claims, taken in conjunction with the accompanying drawings, wherein:

Fig. 1    is a block diagram schematically showing the structure of the cochlear implant according to the present invention;

Fig. 2    is a flow chart showing the whole operation of the cochlear implant according to the present invention;

Fig. 3    is a flow chart showing the operation of channel selection process of the cochlear implant according to the present invention;

Fig. 4    is a flow chart showing the operation of the in- channel stimulating frequency adjusting means of the cochlear implant according to the present invention;

Fig. 5    is a flow chart showing the operation of the inter-channel stimulating frequency adjusting means of the cochlear implant according to the present invention;

Fig. 6    is an explanatory view showing the operation of channel selection process of the cochlear implant according to the present invention;

Fig. 7    is an explanatory view showing the operation of the in-channel stimulating frequency adjusting means of the cochlear implant according to the present invention;

Fig. 8    is an explanatory view showing the operation of the inter-channel stimulating frequency adjusting means of the cochlear implant according to the present invention; and

Fig. 9    is an explanatory view showing the operation of the cochlear implant according to the present invention.

## DESCRIPTION OF THE PREFERRED EMBODIMENTS

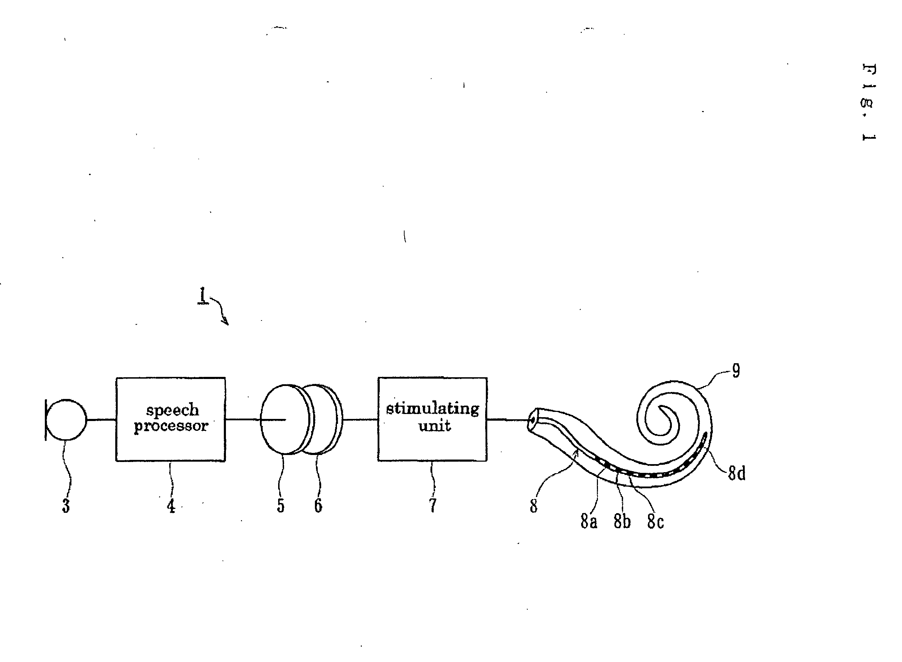

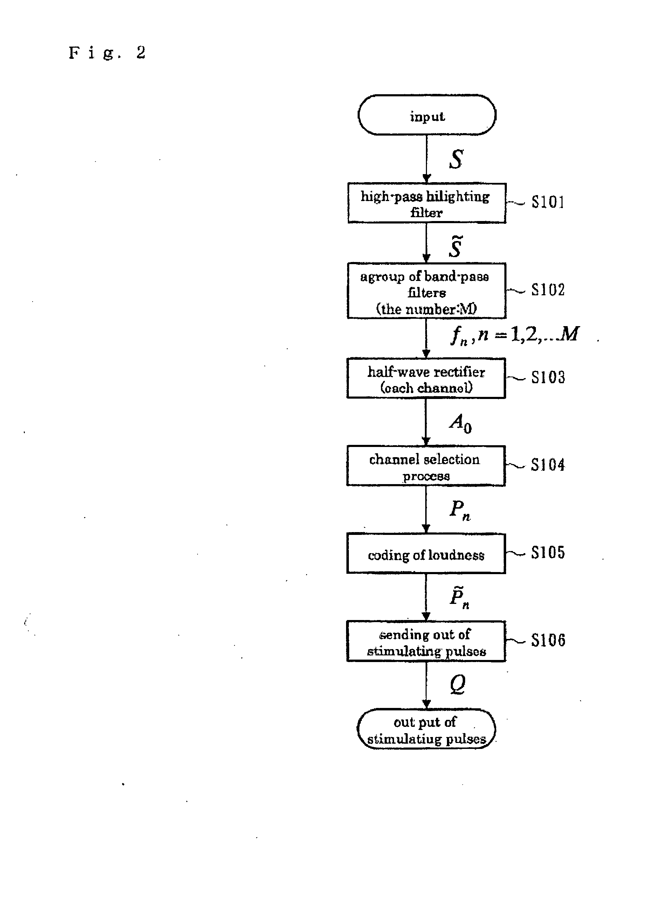

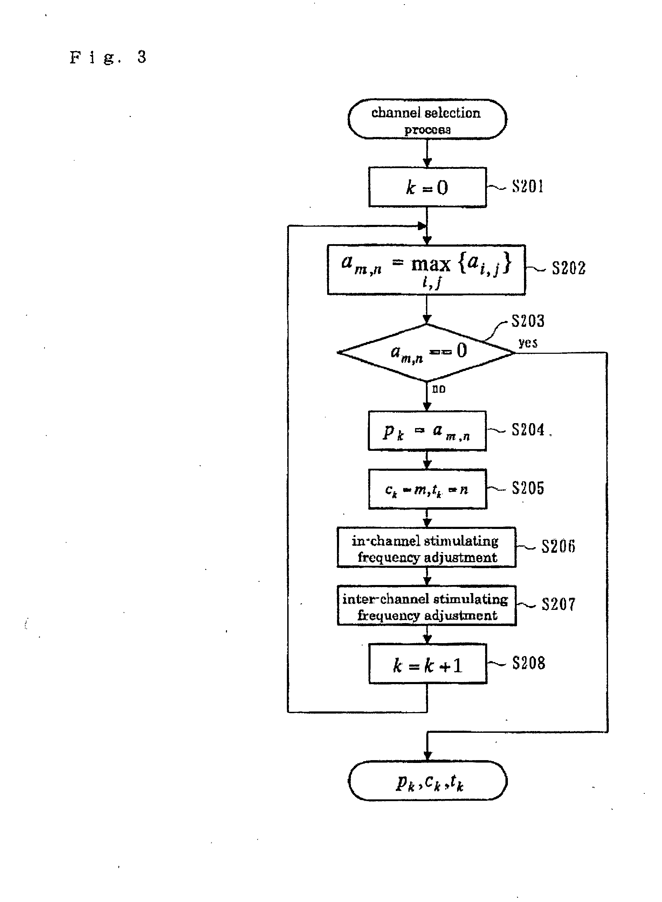

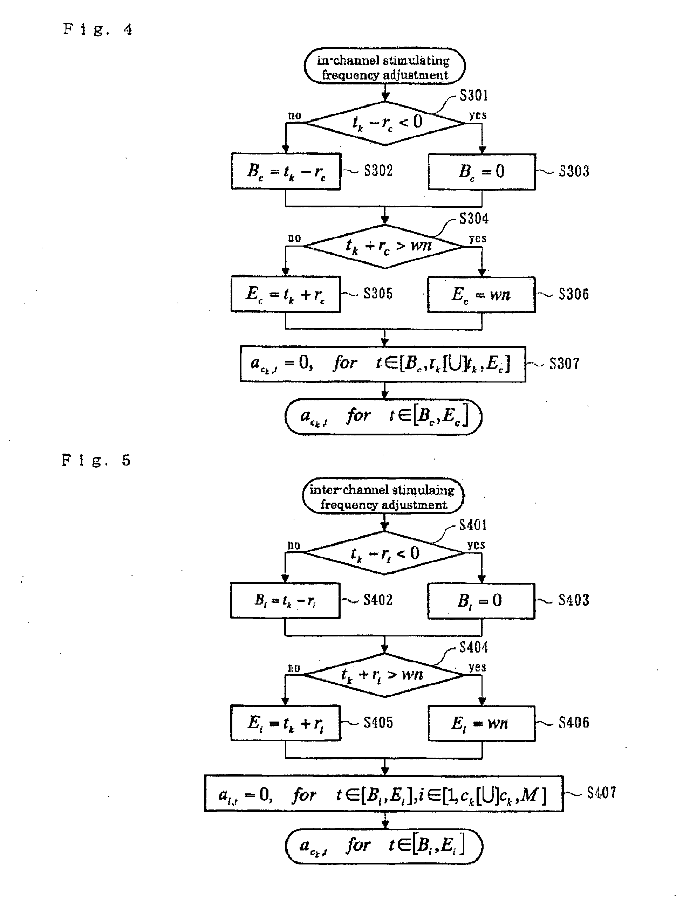

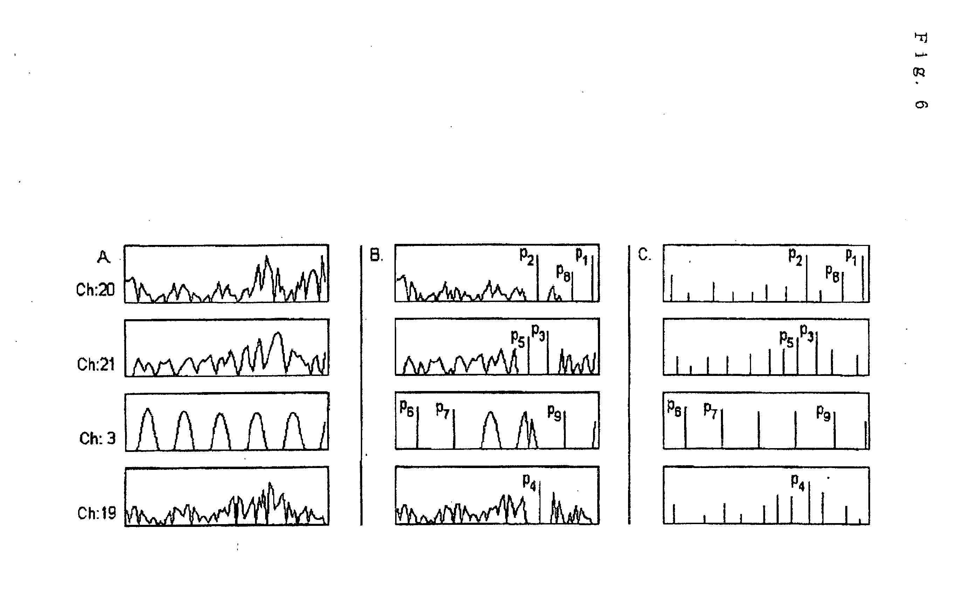

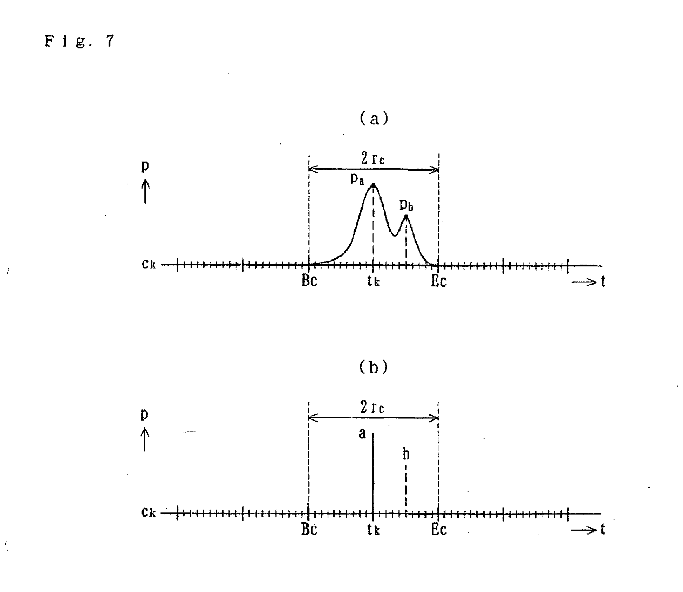

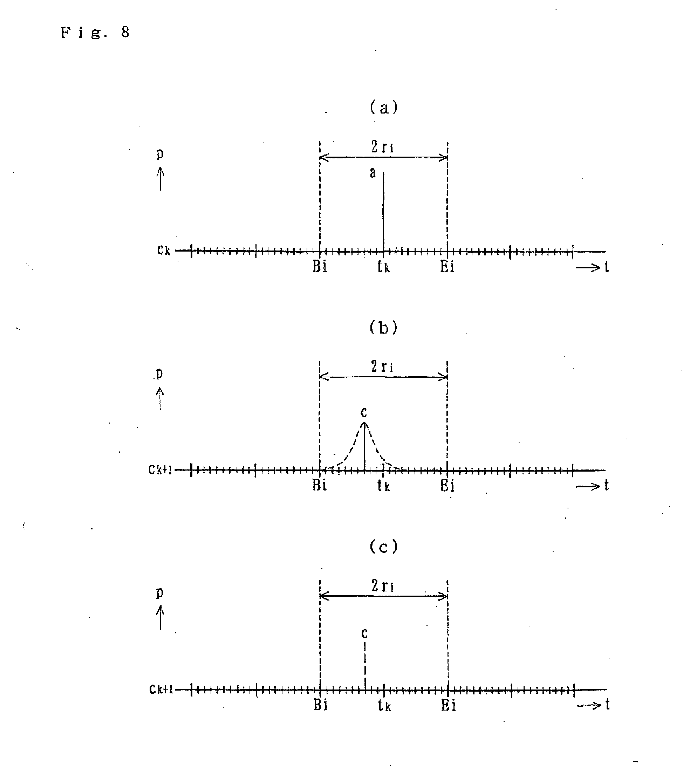

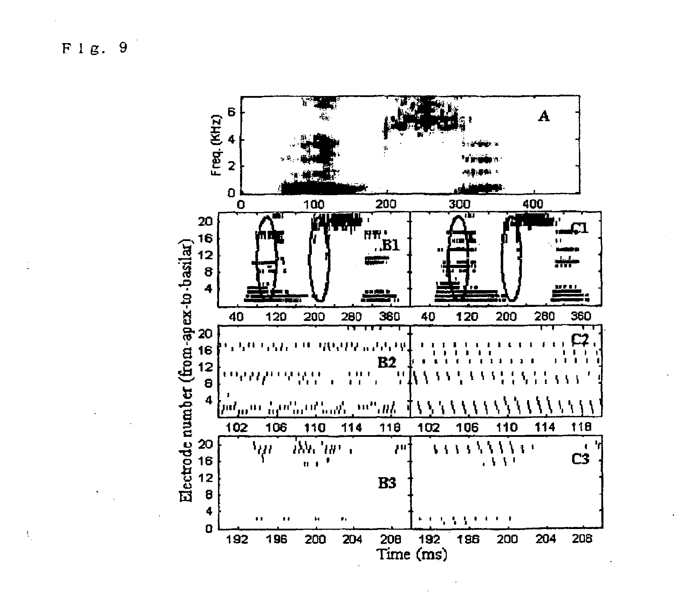

A preferable embodiment of the present invention will described with reference to accompanying drawings. Fig. 1 is a block diagram schematically showing the structure of the cochlear implant according to the present invention; Fig. 2 is a flow chart showing the whole operation of the cochlear implant according to the present invention; Fig. 3 is a flow chart showing the operation of channel selection process of the cochlear implant according to the present invention; Fig. 4 is a flow chart showing the operation of the in-channel stimulating frequency adjusting means of the cochlear implant according to the present invention; Fig. 5 is a flow chart showing the operation of the inter-channel stimulating frequency adjusting means of the cochlear implant according to the present invention; Fig. 6 is an explanatory view showing the operation of channel selection process of the cochlear implant according to the present invention; Fig. 7 is an explanatory view showing the operation of the in-channel stimulating frequency adjusting means of the cochlear implant according to the present invention; Fig. 8 is an explanatory view showing the operation of the inter-channel stimulating frequency adjusting means of the cochlear implant according to the present invention; and Fig. 9 is an explanatory view showing the operation of the cochlear implant according to the present invention.

A cochlear implant 1 is adapted to reinforce the acoustic sense by providing electrically stimulating pulses to the cochlea 9. Concretely, the cochlear implant 1 comprises a microphone 3, a speech processor 4 for programmatically performing the speech process of converting a speech information sampled via the microphone 3 to stimulating pulses, an external coil 5 forming an antenna out of the human body, an internal coil 6 forming an antenna within the human body, a stimulating unit 7 for converting the speech information sent from the speech processor 4 via the external and internal coils 5 and 6 to the stimulating pulses, and an electrode array 8 consisting of a plurality of electrodes 8a ~ 8d arranged within a cochlea for actually outputting the stimulating pulses. The speech can be sensed by the human acoustic nerve with its end being stimulated by an electric current generated by the electrodes 8a ~ 8d.

The electrodes 8a ~ 8d of the electrode array 8 actually include about 22 electrodes and each of them being adapted to be used based on the condition of a user. A whole audible speech range is divided into M bands each forming a channel and each of the electrodes 8a ~ 8d corresponds to one of the channels. Although the illustrated embodiment of the present invention is described based on an example using 4 (four) electrodes 8a ~ 8d, the present invention is not limited to the illustrated embodiment. According to one concrete example of the electrodes 8a ~ 8d, the electrode 8a is a 21-th electrode (channel (Ch.) 21) having its center frequency of about 6226 Hz, the electrode 8b is a 20-th electrode (channel (Ch.) 20) having its center frequency of about 5314 Hz, the electrode 8c is a 19-th electrode (channel (Ch.) 19) having its center frequency of about 907.1 Hz, and the electrode 8d is a 3-rd electrode (channel (Ch.) 3) having its center frequency of about 466.5 Hz.

The operation of the cochlear implant 1 according to the present invention will be hereinafter describe. In the description, bracketed expressions such as "(S101)", "(S102)".....,  correspond to those shown in flow charts of Fig. 2 - Fig. 5.

First of all, whole operation of the cochlear implant of the present invention will be described. A signal S picked up by the microphone 3 is reshaped by a high-pass highlighting filter (S101), and then is passed through a group of band-pass filter (S102). In this group of band-pass filters, the signal S is discriminated into respective frequency bands and the information of channel of corresponding frequency band is added to the speech information. That is, the group of band-pass filers form a channel information adding means. Then the signal S is regulated to a desired data style by half-wave rectifiers of the respective channels (S103) to generate a speech information of integral combination of channel information, time information and signal level information which is written as an amplitude-time matrix (ATM). This is expressed as a following mathematical expression (1) in which I and M are numbers of channel, and T and N are time information in which their speech information appear.

$$A_0 = \begin{bmatrix} a_{1,1} & a_{1,2} & \cdots & a_{1,N} \\ a_{2,1} & a_{2,2} & \cdots & a_{2,N} \\ \vdots & \vdots & \vdots & \vdots \\ a_{M,1} & a_{M,2} & \cdots & a_{M,N} \end{bmatrix} \quad I = \{1,2,3,...,M\} \quad T = \{1,2,3,...,N\} \tag{1}$$

The speech information written as the ATM is within a signal frame being a predetermined and fixed sampling period and following processes are successively repeated in respective signal frames. The length of the signal frame in the preferred embodiment of the present invention is about 10 mS, however this is not limited to 10 mS. The length of the signal frame should be determined in consideration of facts that the regeneration of the speech information will be delayed if the length of the signal frame is too long, on the contrary, the speech information cannot be detected if it is shorter than the fundamental period of speech (a period of vibration of the vocal cord).

Then a channel selection process for the speech process to extract the speech information necessary for the actually outputted stimulating pulses from the speech information of the ATM (S104). The detail of the channel selection process will be hereinafter described in more detail. Then the signal level (including the electric current level) is adjusted so that the speech information obtained by the channel selection process becomes stimulating pulses which can provide a suitable sound feeling (loudness) in accordance with the dynamic range of the stimulating pulses (S105). The obtained speech information is sent to the stimulating unit 7 and the stimulating pulses are outputted from each of the electrodes 8a ~ 8d (S106).

Then the detailed process of the channel selection process (S104) will be described. First of all, a counter "k" in the channel selection process is reset (S201). Then the speech information in the ATM are put in order of magnitude of the signal level (S202). At this point of time, if the maximum value is zero (0), no more process is required and thus the process is completed (S203: yes).

The highest signal level of the speech information, the information of channel, and the information of the time are put into variables $p_k$, $c_k$, and $t_k$ respectively. (S204 and S205). A mathematical expression (2) for finding out the maximum value and put it into the variable $p_k$ is shown as follows:

$$a_{m,n} = \max_{i,j} \left\{ a_{i,j} \right\}, \, p_k = a_{m,n} , \, i \in I, \, j \in T \tag{2}$$

Then an in-channel stimulating frequency adjustment is carried out for adjusting the number of the stimulating pulses to a frequency (stimulating rate) allowed in one channel by leaving in respective channels within the signal frame a speech information having a higher signal level in one channel (S206).

The in-channel stimulating frequency adjustment will be described with reference to Fig. 7. Fig. 7 (a) is a graph having the abscissa (time: t) and the ordinate (signal level: p) and shows a speech information before process of the channel $k(c_k)$. The scale of the abscissa indicates the sampling rate. That is, the scale unit of the abscissa indicates the presence of the speech information before treatment. The sampling rate is for example 20 kHz. A symbol "$r_c$" is the inverse number of the channel stimulating rate Rc (e.g. 1800 pps) and indicates a period in which the presence of only one stimulating pulse is allowed. The channel stimulating rate Rc is a stimulating rate per one channel.

In one time in-channel stimulating frequency adjustment, it should be processed during a period from a point of time ($B_c$) before from a point of time $t_k$ by a period $r_c$, to a point of time ($E_c$) after from a point of time $t_k$ by a period $r_c$. $B_c$ is determined as a time which is obtained by subtracting $r_c$ from $t_k$ (S302), if the value obtained by subtracting $r_c$ from $t_k$ is larger than zero (0) (S301: no) and is also determined as zero (0) as a head of the signal frame (S303), if the value obtained by subtracting $r_c$ from $t_k$ is smaller than zero (0) (S301: yes). $E_c$ is determined as a time which is obtained by adding $r_c$ to $t_k$ (S305), if the value obtained by adding $r_c$ to $t_k$ is smaller than a last time (wn) of the signal frame (S304: no) and is also determined as wn as a tail end of the signal frame (S306), if the value obtained by adding $r_c$ to $t_k$ is larger than last time (wn) (S304: yes).

A process for leaving the speech information having a highest signal level is carried out during a period from $B_c$ to $E_c$. This will be described with reference to Fig. 7. As shown in Fig. 7 (a), only the speech information $p_a$ having

the maximum value is left and the other speech information should be abandoned although there are two or more speech information $p_a$ and $p_b$ having a higher signal level during the period from $B_c$ to $E_c$. That is, as shown in Fig. 7 (b), it is only the signal "a" to be left during the period from $B_c$ to $E_c$. A following mathematical expression (3) is used to leave the maximum value (S307).

$$a_{c_k,t} = 0, \quad for \quad t \in [B_c, t_k[ \bigcup ]t_k, E_c] \qquad (3)$$

The in-channel stimulating frequency adjustment can be expressed by a following mathematical expression (4).

$$a_{m,n} = 0, where \quad \left| n - n_c \right| \leq \frac{1}{R_c}, n \in T \qquad (4)$$

Then inter-channel stimulating frequency adjustment (S207) is carried out for adjusting so that the number of the stimulating pulses should be a frequency (impulse stimulating rate) allowed in the whole channel by leaving speech information having a high signal level in the signal frame.

The inter-channel stimulating frequency adjustment will be described with reference to Fig. 8. Fig. 8 (a) is a graph having the abscissa (time: t) and the ordinate (signal level: p) and shows a speech information after the in-channel stimulating frequency adjustment of the channel $k(c_k)$. The scale of the abscissa indicates the sampling rate. That is, the scale unit of the abscissa indicates the presence of the speech information before treatment. The sampling rate is for example 20 kHz. A symbol "$r_i$" is the inverse number of the impulse stimulating rate Ri (e.g. 14400 pps) and indicates a period in which the presence of only one stimulating pulse is allowed. The impulse stimulating rate Ri is a stimulating rate per one signal frame considering all channels.

In one time inter-channel stimulating frequency adjustment, it should be processed during a period from a point of time ($B_i$) before from a point of time $t_k$ by a period $r_i$, to a point of time ($E_i$) after from a point of time $t_k$ by a period $r_i$. $B_i$ is determined as a time which is obtained by subtracting $r_i$ from $t_k$ (S402), if the value obtained by subtracting $r_i$ from $t_k$ is larger than zero (0) (S401: no) and is also determined as zero (0) as a head of the signal frame (S403), if the value obtained by subtracting $r_i$ from $t_k$ is smaller than zero (0) (S401: yes). $E_i$ is determined as a time which is obtained by adding $r_i$ to $t_k$ (S405), if the value obtained by adding $r_i$ to $t_k$ is smaller than a last time (wn) of the signal frame (S404: no) and is also determined as (wn) as a tail end of the signal frame (S406), if the value obtained by adding $r_i$ to $t_k$ is larger than last time (wn) (S404: yes).

A process for leaving the speech information having a highest signal level is carried out during a period from $B_i$ to Ei. This will be described with reference to Fig. 8. As shown in Figs. 8 (a) and 8 (b), when there is a signal "a" in a period from $B_i$ to $E_i$ at a channel $c_k$ and a signal "c" at a channel $c_{k+1}$, only the signal "a" having a higher signal level is left and the other speech information should be abandoned. That is, as shown in Fig. 8 (c), it is only the signal "a" to be left during the period from $B_i$ to $E_i$. A following mathematical expression (5) is used to leave the maximum value (wherein $R_c < R_i$) (S407).

$$a_{i,t} = 0, \quad for \quad t \in [B_i, E_i], i \in [1, c_k[ \bigcup ]c_k, M] \qquad (5)$$

The inter-channel stimulating frequency adjustment can be expressed by a following mathematical expression (6).

$$a_{l,n} = 0, where \quad \left| n - n_i \right| \le \frac{1}{R_i}, l \in I, n \in T \qquad (6)$$

After this time, the processes (S202 ~ S207) stated above are repeated in one signal frame (S208). If the maximum value is zero (0) at a next period $t_k$, no more process is required and thus the process is completed (S203: yes). From these processes, the information $t_k$ of time is put into signal level $p_k$ of speech information and the information $c_k$ of channel in respective counter k and is outputted from the channel selection process (S104).

Whole of the channel selection processes (S104) is shown in Fig. 6. Fig. 6A shows the speech information before processes of four (4) channels. As shown in Fig. 6B, a region near the signal $p_1$ having a highest signal level in the signal frame is firstly removed and then same process is carried on as to a signal $p_2$ and thus same process is carried out successively in the same channel. Such a process is also carried out in other channels. Only speech information necessary for the stimulating pulses are finally left as shown in Fig. 6C.

Fig. 9 shows results of an actual speech process in which a speech "utsu" is processed. Fig 9A is a spectrogram before process in which B1 ~ B3 show outputs of results speech processed in accordance with the present invention. On the contrary, C1 ~ C3 show results processed by a conventional technology in comparison with the results of B1 ~ B3. In B1 ~ B3 (C1 ~ C3), B1 (C1) shows a whole output of the signal frame and B2 (C2) is an enlargement of a front vertical ellipse and shows a portion "u" of the speech "utsu". On the other hand, B3 (C3) is an enlargement of a central vertical ellipse and shows a portion "ts" of the speech "utsu".

Heretofore since it has been thought that it is preferable to output all sampled speech information as stimulations without omission in order to regenerate the speech more faithfully to the actual speech, some of simultaneously generated speech information have been outputted purposely later than other simultaneously generated speech information. However this delay causes unnatural speech regeneration. This unnatural condition is apparent from a fact that the outputs shown in C2 and C3 in Fig. 9 have become regular outputs. According to the present invention, the periodicity of the outputs of stimulating pulses in each channel can be obtained at a relatively high probability and it corresponds to the inverse number of the center frequency of corresponding channel.

The inventors of the present invention have found that sufficiently comprehensible regeneration of speech can be obtained by outputting only selected speech information having a high signal level without outputting all sampled speech information as stimulations without omission. In addition, according to the speech processing method of the present invention, it is possible to provide wearers of the cochlear implant with speech near the natural speech since the relative time interval of the speech information to be regenerated can be kept.

According to the cochlear implant 1 of the present invention, it is possible to regenerate natural variation of respective channels at a value near the natural period and at relatively high probability since only the speech information having the highest signal level within the period in which presence of only one stimulating pulse is allowed by in-channel and inter-channel stimulating frequency adjustments.

Although it is described with reference to the preferred embodiment of the present invention that the adjustment of stimulation is carried out every one signal frame, it is also possible to carry out the stimulation adjustment in consideration of other signal frames.

According to the present invention of claim 1, it is possible to provide wearers of the cochlear implant with speech near the natural speech since the same order as those of the generation of the speech information as well as the relative time interval of the speech information to be regenerated can be kept because of the speech information having high signal level being left in order to adjust the number of the stimulating pulses to be the allowed stimulation.

According to the present invention, it is possible to regenerate natural variation of respective channels at a value near the natural period and at relatively high probability since only the speech information having the highest signal level within the period in which presence of only one stimulating pulse is allowed.

According to claim 2 of the present invention, it is possible to provide wearers of the cochlear implant with speech near the natural speech since the same order as those of the generation of the speech information as well as the relative time interval of the speech information to be regenerated can be kept because of the speech information having high signal level being left in order to adjust the number of the stimulating pulses to be the allowed stimulation.

According to the present invention, it is possible to regenerate natural variation of respective channels at a value near the natural period and at relatively high probability since only the speech information having the highest signal level within the period in which presence of only one stimulating pulse is allowed.

The present invention has been described with reference to the preferred embodiment. Obviously, modifications and alternations will occur to those of ordinary skill in the art upon reading and understanding the preceding detailed description. It is intended that the present invention be construed as including all such alternations and modifications insofar as they come within the scope of the appended claims.

**Claims**

1. A method of speech conversion for a cochlear implant (1) in which an electrode array (8) of a plurality of electrodes is to be arranged within a cochlea (9), electrically stimulating pulses are generated by transmitting speech information sampled via a microphone (3) to the electrodes, and the acoustic sense is reinforced by transmitting the speech information to the cochlea as electrically stimulating pulses, the method comprising steps of:

   - discriminating (S102) the speech information into respective channels using a plurality of band pass filters within a signal frame being a predetermined sampling period,
   - **characterized by**
   - adjusting (S206) the number of the stimulating pulses to a number allowed in each channel by leaving in each channel the speech information having the highest signal level and abandoning other speech information in each channel within a period in wich presence of only stimulating pulse in one channel is allowed,
   - adjusting (S207) the number of the stimulating pulses to a number allowed in all channels by leaving a speech information having the highest signal level of all speech information left in all channels and abandoning other speech information left in all channels within a period in wich presence of only stimulating pulse in all channels isallowed and
   - generating (S106) the stimulating pulses by providing the speech information left in all channels to the electrode corresponding to the channel of the left speech information.

2. A cochlear implant (1) comprising an electrode array (8) of a plurality of electrodes to be arranged within a cochlea (9), wherein the cochlear implant is designed to generate electrically stimulating pulses by transmitting speech information sampled via a microphone (3) to the electrodes in order to reinforce the acoustic sense by transmitting the speech information to the cochlea as electrically stimulating pulses, the cochlear implant comprising:

   - a plurality of band pass filters for discriminating speech information into respective channels, **characterised by,**
   - an in-channel stimulating frequency adjusting means for adjusting the number of the stimulating pulses to a number allowed in each channel by leaving in each channel the speech information having the highest signal level within the signal frame and abandoning other speech information in each channel within a period in wich presence of only one stimulating pulse in one channel is allowed,
   - an inter-channel stimulating frequency adjusting means for adjusting the number of the stimulating pulses to a number allowed in all channels by leaving the speech information having the highest signal level of all speech information left in all channels and abandoning other speech information left in all channels within a period in which presence of only one stimulating pulse in all channels is allowed and
   - a stimulating pulse generating means for converting the speech information left in all channels to the stimulating pulse and providing the stimulating pulse to the electrode corresponding to the channel of the left speech information.
   within a period in which presence of only one stimulating pulse in one channel is allowed

**Patentansprüche**

1. Verfahren zur Sprachumwandlung für ein Cochlear-Implantat (1), bei dem eine Elektrodenanordnung (8) aus mehreren Elektroden in einer Cochlea (9) angeordnet werden soll, elektrisch stimulierende Impulse durch Übertragen von über ein Mikrofon (3) abgetasteten Sprachinformationen zu den Elektroden erzeugt werden und der Hörsinn durch Übertragen der Sprachinformation zur Cochlea als elektrisch stimulierende Impulse verstärkt wird, wobei das Verfahren die folgenden Schritte beinhaltet:

   - Aufteilen (S102) der Sprachinformationen auf jeweilige Kanäle mittels mehrerer Bandpassfilter innerhalb eines Signalrahmens, der eine vorbestimmte Abtastperiode ist, **gekennzeichnet durch**
   - Anpassen (S206) der Anzahl der Stimulierungsimpulse an eine Zahl, die in jedem Kanal zulässig ist, indem in jedem Kanal die Sprachinformation mit dem höchsten Signalpegel belassen und andere Sprachinformationen in jedem Kanal innerhalb einer Periode verworfen werden, in der die Anwesenheit von nur einem stimulierenden Impuls in einem Kanal zulässig ist,
   - Anpassen (S207) der Anzahl der Stimulierungsimpulse an eine in allen Kanälen zulässige Zahl, indem eine Sprachinformation mit dem höchsten Signalpegel aller in allen Kanälen belassenen Sprachinformationen belassen und andere in allen Kanälen belassenen Sprachinformationen innerhalb einer Periode, in der die Anwesenheit von nur einem Stiumlierungsimpuls in allen Kanälen zulässig ist, verworfen werden, und

- Erzeugen (S106) der Stimulierungsimpulse **durch** Übertragen der in allen Kanälen belassenen Sprachinformationen zu der Elektrode, die dem Kanal der belassenen Sprachinformation entspricht.

**2.** Cochlear-Implantat (1), das eine Elektrodenanordnung (8) aus mehreren in einer Cochlea (9) anzuordnenden Elektroden umfasst, wobei das Cochlear-Implantat so ausgelegt ist, dass es elektrisch stimulierende Impulse durch Übertragen von über ein Mikrofon (3) abgetasteten Sprachinformationen zu den Elektroden erzeugt, um den Hörsinn durch Übertragen der Sprachinformation zur Cochlea als elektrisch stimulierende Impulse zu verstärken, wobei das Cochlear-Implantat Folgendes umfasst:

- mehrere Bandpassfilter zum Aufteilen von Sprachinformation auf jeweilige Kanäle, **gekennzeichnet durch**:
- ein In-Kanal-Stimulationsfrequenzanpassmittel zum Justieren der Anzahl der Stimulierungsimpulse an eine in jedem Kanal zulässige Zahl, indem in jedem Kanal die Sprachinformation mit dem höchsten Signalpegel in dem Signalrahmen belassen und andere Sprachinformationen in jedem Kanal innerhalb einer Periode verworfen werden, in der die Anwesenheit von nur einem stimulierenden Impuls in einem Kanal zulässig ist,
- ein Zwischen-Kanal-Stimulationsfrequenzanpassmittel zum Anpassen der Anzahl der Stimulierungsimpulse an eine in allen Kanälen zulässige Zahl, indem die Sprachinformation mit dem höchsten Signalpegel aller in allen Kanälen belassenen Sprachinformationen belassen und andere in allen Kanälen belassene Sprachinformationen innerhalb einer Periode, in der die Anwesenheit von nur einem Stiumlierungsimpuls in allen Kanälen zulässig ist, verworfen werden, und
- ein Stimulierungsimpulserzeugungsmittel zum Umwandeln der in allen Kanälen belassenen Sprachinformation in den Stimulierungsimpuls und Übertragen des Stimulierungsimpulses zu der Elektrode, die dem Kanal der belassenen Sprachinformation entspricht.

## Revendications

**1.** Procédé de conversion vocale pour un implant cochléaire (1) dans lequel un ensemble d'électrodes (8) d'une pluralité d'électrodes est destiné à être agencé à l'intérieur d'une cochlée (9), des impulsions de stimulation électrique sont générées en transmettant des informations vocales échantillonnées via un microphone (3) aux électrodes, et le sens auditif est renforcé en transmettant les informations vocales à la cochlée en tant qu'impulsions de stimulation électrique, le procédé comprenant les étapes consistant à :

- différencier (S102) les informations vocales dans des canaux respectifs à l'aide d'une pluralité de filtres passe-bande à l'intérieur d'une trame de signal qui est une période d'échantillonnage prédéterminée, **se caractérisant par**
- l'ajustement (S206) du nombre d'impulsions de stimulation à un nombre autorisé dans chaque canal en laissant dans chaque canal l'information vocale ayant le niveau de signal le plus élevé et en abandonnant les autres informations vocales dans chaque canal à l'intérieur d'une période dans laquelle la présence d'une seule impulsion de stimulation dans un canal est autorisée,
- l'ajustement (S207) du nombre des impulsions de stimulation à un nombre autorisé dans tous les canaux en laissant une information vocale ayant le niveau de signal le plus élevé de toutes les informations vocales laissées dans tous les canaux et en abandonnant les autres informations vocales laissées dans tous les canaux à l'intérieur d'une période dans laquelle la présence d'une seule impulsion de stimulation dans tous les canaux est autorisée et
- la génération (S106) des impulsions de stimulation en fournissant les informations vocales laissées dans tous les canaux à l'électrode correspondant au canal des informations vocales laissées.

**2.** Implant cochléaire (1) comprenant un réseau d'électrodes (8) d'une pluralité d'électrodes à agencer à l'intérieur d'une cochlée (9), dans lequel l'implant cochléaire est conçu pour générer des impulsions de stimulation électrique en transmettant des informations vocales échantillonnées via un microphone (3) aux électrodes afin de renforcer le sens auditif en transmettant les informations vocales à la cochlée en tant qu'impulsions de stimulation électrique, l'implant cochléaire comprenant:

- une pluralité de filtres passe-bande destinés à différencier les informations vocales dans des canaux respectifs, **se caractérisant par**
- un moyen d'ajustement de fréquence de stimulation dans les canaux destiné à ajuster le nombre des impulsions de stimulation à un nombre autorisé dans chaque canal en laissant dans chaque canal l'information vocale ayant le niveau de signal le plus élevé à l'intérieur de la trame de signal et en abandonnant les autres informations vocales dans chaque canal à l'intérieur d'une période dans laquelle la présence d'une seule impulsion de stimulation dans un canal est autorisée,

- un moyen d'ajustement de fréquence de stimulation entre les canaux destiné à ajuster le nombre des impulsions de stimulation à un nombre autorisé dans tous les signaux en laissant l'information vocale ayant le niveau de signal le plus élevé de toutes les informations vocales laissées dans tous les canaux et en abandonnant les autres informations laissées dans tous les canaux à l'intérieur d'une période dans laquelle la présence d'une seule impulsion de stimulation dans tous les canaux est autorisée et

- un moyen de génération d'impulsions de stimulation destiné à convertir les informations vocales laissées dans tous les canaux en l'impulsion de stimulation et à fournir l'impulsion de stimulation à l'électrode correspondant au canal des informations vocales laissées.

EP 1 652 498 B1

Fig. 1

9
8d
8c
8b
8a
8

stimulating
unit
7

6
5

1

speech
processor
4

3

10

Ｆ ｉ ｇ. 2

```
        ╭──────────────╮
        │    input     │
        ╰──────────────╯
               │ $S$
               ▼
   ┌──────────────────────┐
   │ high-pass hilighting  │── S101
   │       filter          │
   └──────────────────────┘
               │ $\tilde{S}$
               ▼
   ┌──────────────────────┐
   │ agroup of band-pass   │── S102
   │       filters         │
   │    (the number:M)     │
   └──────────────────────┘
               │ $f_n, n = 1, 2, ... M$
               ▼
   ┌──────────────────────┐
   │  half-wave rectifier  │── S103
   │    (each channel)     │
   └──────────────────────┘
               │ $A_0$
               ▼
   ┌──────────────────────┐
   │  channel selection    │── S104
   │       process         │
   └──────────────────────┘
               │ $P_n$
               ▼
   ┌──────────────────────┐
   │  coding of loudness   │── S105
   └──────────────────────┘
               │ $\tilde{P}_n$
               ▼
   ┌──────────────────────┐
   │   sending out of      │── S106
   │  stimulating pulses   │
   └──────────────────────┘
               │ $Q$
               ▼
        ╭──────────────╮
        │   out put of  │
        │ stimulatiug pulses │
        ╰──────────────╯
```

EP 1 652 498 B1

F i g. 3

channel selection
process

$k = 0$ — S201

$a_{m,n} = \max_{i,j} \{a_{i,j}\}$ — S202

$a_{m,n} == 0$ — S203
yes

no

$p_k = a_{m,n}$ — S204

$c_k = m, t_k = n$ — S205

in-channel stimulating
frequency adjustment — S206

inter-channel stimulating
frequency adjustment — S207

$k = k + 1$ — S208

$p_k, c_k, t_k$

F i g. 4

$$\text{in-channel stimulating frequency adjustment}$$

S301

$$t_k - r_c < 0$$

no        yes

$$B_c = t_k - r_c \quad \sim S302 \qquad\qquad B_c = 0 \quad \sim S303$$

S304

$$t_k + r_c > wn$$

no        yes

$$E_c = t_k + r_c \quad \sim S305 \qquad\qquad E_c = wn \quad \sim S306$$

$$a_{c_k,t} = 0, \quad for \quad t \in [B_c, t_k[\cup]t_k, E_c] \quad \sim S307$$

$$a_{c_k,t} \quad for \quad t \in [B_c, E_c]$$

F i g. 5

$$\text{inter-channel stimulating frequency adjustment}$$

S401

$$t_k - r_i < 0$$

no        yes

$$B_i = t_k - r_i \quad \sim S402 \qquad\qquad B_i = 0 \quad \sim S403$$

S404

$$t_k + r_i > wn$$

no        yes

$$E_i = t_k + r_i \quad \sim S405 \qquad\qquad E_i = wn \quad \sim S406$$

$$a_{i,t} = 0, \quad for \quad t \in [B_i, E_i], i \in [1, c_k[\cup]c_k, M] \quad \sim S407$$

$$a_{c_k,t} \quad for \quad t \in [B_i, E_i]$$

F i g . 6

F i g. 7

( a )

( b )

Fig. 8

(a)

(b)

(c)

F i g. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Philipos L Loizou.** Mimicking the human ear: An Overview of Signal-Processing Strategies for converting sound into Electrical signals in Cochlear Implants. *IEEE SIGNAL PROCESSING MAGAZINE,* September 1998